(19) Europäisches Patentamt
European Patent Office
Office européen des brevets

(11) Veröffentlichungsnummer: **0 166 939**
**B1**

# (12) EUROPÄISCHE PATENTSCHRIFT

(45) Veröffentlichungstag der Patentschrift: **22.06.88**

(51) Int. Cl.⁴: **C 07 D 401/04**, A 61 K 31/495 // C07D215/56

(21) Anmeldenummer: **85106254.7**

(22) Anmeldetag: **22.05.85**

## (54) 1-Cyclopropyl-6-fluor-1,4-dihydro-4-oxo-7-(3-oxo-1-piperazinyl)-3-chinolincarbonsäuren, Verfahren zu ihrer Herstellung sowie diese enthaltende antibakterielle Mittel.

(30) Priorität: **04.06.84 DE 3420770**

(43) Veröffentlichungstag der Anmeldung: **08.01.86 Patentblatt 86/2**

(45) Bekanntmachung des Hinweises auf die Patenterteilung: **22.06.88 Patentblatt 88/25**

(84) Benannte Vertragsstaaten: **AT BE CH DE FR GB IT LI NL SE**

(56) Entgegenhaltungen:
**EP - A - 0 049 355**
**FR - A - 2 437 406**
**US - A - 4 398 029**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Petersen, Uwe, Dr., Auf dem Forst 4, D-5090 Leverkusen 1 (DE)**
Erfinder: **Grohe, Klaus, Dr., Am Wasserturm 10, D-5068 Odenthal (DE)**
Erfinder: **Zeiler, Hans-Joachim, Dr., Elsbeeker Strasse 46, D-5620 Velbert 15 (DE)**
Erfinder: **Metzger, Karl Georg, Dr., Pahlkestrasse 75, D-5600 Wuppertal 1 (DE)**

EP 0 166 939 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 1-Cyclopropyl-6-fluor-1,4-dihydro-4-oxo-7-(3-oxo-1-piperazinyl)-3-chinolincarbonsäuren, Verfahren zu ihrer Herstellung sowie diese enthaltende antibakterielle Mittel und Futterzusatzstoffe.

Es wurde gefunden, dass die neuen 1-Cyclopropyl-6-fluor-1,4-dihydro-4-oxo-7-(3-oxo-1-piperazinyl)-3-chinolincarbonsäuren der Formel (I)

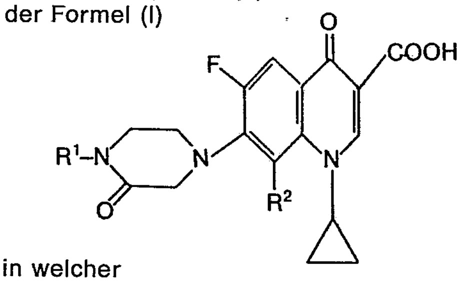

(I),

in welcher
$R^1$ Wasserstoff, Methyl oder Ethyl und
$R^2$ Wasserstoff oder Fluor bedeuten,
sowie deren pharmazeutisch verwendbaren Hydrate, Alkali- und Erdalkalisalze eine hohe antibakterielle Wirkung aufweisen.

Weiterhin wurde gefunden, dass man die erfindungsgemässen Verbindungen der Formel (I) erhält, wenn man eine Verbindung der Formel (II)

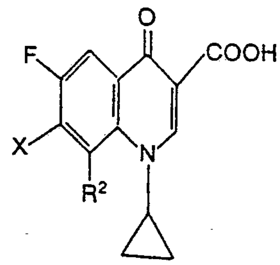

(II),

in welcher
$R^2$ die oben angegebene Bedeutung hat und
X für Chlor, Brom oder Fluor steht,
mit einem 2-Piperazinon der Formel (III),

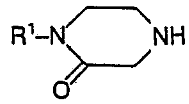

(III)

in welcher
$R^1$ die oben angegebene Bedeutung hat, umsetzt.

Überraschenderweise zeigen die erfindungsgemässen 1-Cyclopropyl-6-fluor-1,4-dihydro-4-oxo-7-(3-oxo-1-piperazinyl)-3-chinolincarbonsäuren eine höhere antibakterielle Wirkung als die nach dem Stand der Technik bekannte 1-Ethyl-6-fluor-1,4-dihydro-4-oxo-7-(1-piperazinyl)-3-chinolincarbonsäure.

Sie eignen sich daher als Wirkstoffe für die Human- und Veterinärmedizin, wobei zur Veterinärmedizin auch die Behandlung von Fischen zur Therapie und Vorbeugung bakterieller Infektionen zu zählen ist.

Die erfindungsgemässen Stoffe stellen somit eine Bereicherung der Pharmazie dar.

Verwendet man beispielsweise 1-Cyclopropyl-6,7-difluor-1,4-dihydro-4-oxo-3-chinolincarbonsäure und 2-Piperazinon als Ausgangssubstanzen, so kann der Reaktionsablauf durch das folgende Formelschema wiedergegeben werden:

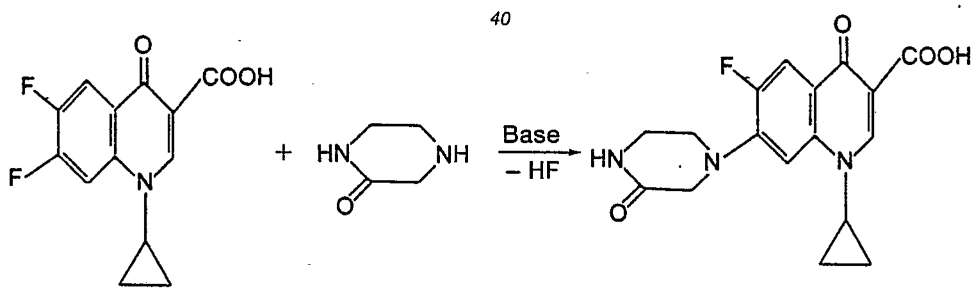


Die als Ausgangsverbindungen verwendeten Chinoloncarbonsäuren der Formel (II) sind teilweise bekannt (vgl. DE-OS 3 142 854 = 7-Chlor-1-cyclopropyl-6-fluor-1,4-dihydro-4-oxo-3-chinolincarbonsäure) oder lassen sich auf folgendem Weg herstellen:

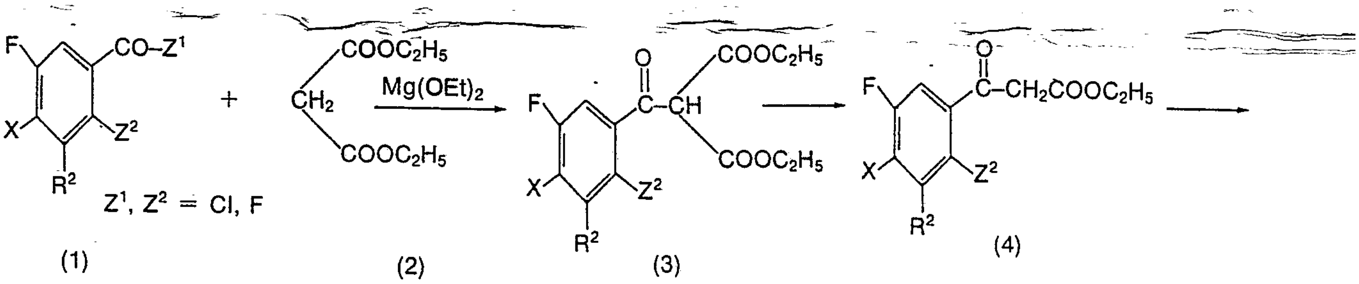


(1) (2) (3) (4)

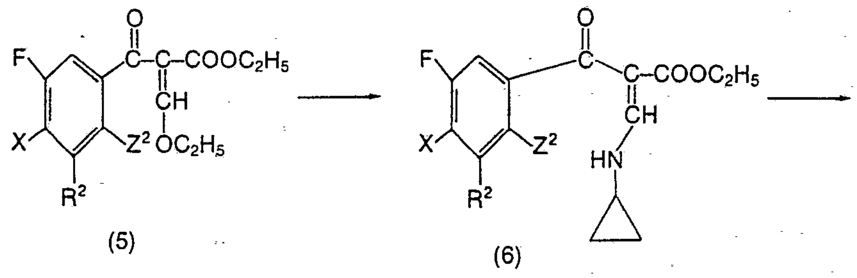

(5) (6)

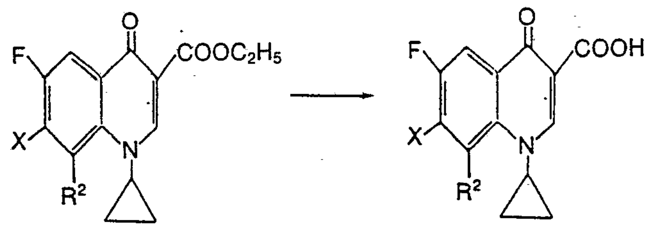

(7) (II)

Danach wird Malonsäurediethylester (2) in Gegenwart von Magnesiumethylat mit dem entsprechenden Benzoylfluorid bzw. -chlorid (1) zum Aroylmalonester (3) acyliert (Organicum, 3. Aufl. 1964, S. 438).

Durch partielle Verseifung und Decarboxylierung von (3) in wässrigem Medium mit katalytischen Mengen Schwefelsäure oder p-Toluolsulfonsäure erhält man in guter Ausbeute den Aroylessigsäureethylester (4), der mit Orthoameisensäure-triethylester/Acetanhydrid in den entsprechenden 2-Benzoyl-3-ethoxy-acrylsäureethylester (5) übergeht. Die Umsetzung von (5) mit Cyclopropylamin in einem Lösungsmittel, wie z.B. Methylenchlorid, Ethanol, Chloroform, Cyclohexan oder Toluol führt in leicht exothermer Reaktion zum gewünschten Zwischenprodukt (6).

Die Cyclisierungsreaktion (6) → (7) wird in einem Temperaturbereich von etwa 60 bis 300 °C, bevorzugt 80 bis 180 °C durchgeführt.

Als Verdünnungsmittel können Dioxan, Dimethylsulfoxid, N-Methylpyrrolidon, Sulfolan, Hexamethylphosphorsäuretriamid und bevorzugt N,N-Dimethylformamid verwendet werden.

Als Säurebinder kommen für diese Reaktionsstufe Kalium-tert.-butanolat, Butyl-lithium, Lithium-phenyl, Phenylmagnesiumbromid, Natriummethylat, Natriumhydrid, Natrium- oder Kaliumcarbonat in Betracht. Wenn Fluorwasserstoff abgespalten werden soll ($Z^2$ = F), haben sich auch Kalium- oder Natrium-fluorid als besonders geeignet erwiesen. Es kann vorteilhaft sein, einen Überschuss von 10 Mol-% an Base einzusetzen.

Die in dem letzten Schritt erfolgte Esterhydrolyse von (7) unter basischen oder sauren Bedingungen führt zu 1-Cyclopropyl-6-fluor-7-halogen-1,4-dihydro-4-oxo-3-chinolin-carbonsäure (II).

Das als Ausgangsmaterial für diesen Syntheseweg verwendete 2,3,4,5-Tetrafluorbenzoylchlorid (1) ($R^2$ = X = $Z^2$ = F, $Z^1$ = Cl) wurde aus der literaturbekannten 2,3,4,5-Tetrafluor-benzoesäure [G.G. Yakobson, V.N. Odinokov und N.N. Vorozhtsov Jr., Zh. Obsh. Khim. 36, 139 (1966)] mit Thionylchlorid auf übliche Weise erhalten. Es besitzt einen Siedepunkt von 75–80 °C/17 mbar. Das 2,3,4,5-Tetrafluorbenzoylchlorid besitzt einen Siedepunkt von 46 bis 47 °C/20 mbar ($n_D^{20}$: 1,4375).

Analog wurde das als Ausgangsprodukt verwendete 2,4,5-Trifluorbenzoylchlorid (1) ($R^2$ = H, X = $Z^1$ = $Z^2$ = F) aus der literaturbekannten 2,3,5-Trifluorbenzoesäure [I.J. DeGraw, M. Corey u. W.A. Steiner, J. Chem. Eng. Data 13, 587 (1968)] hergestellt. Es besitzt einen Siedepunkt von 53–56 °C/18 mbar ($n_D^{20}$ = 1,4546).

Die als Ausgangsprodukte verwendeten Piperazinone (III) sind literaturbekannt [S.R. Aspinall, J. Amer. Chem. Soc. 62, 1202 (1940)].

Die Umsetzung von (II) mit (III) wird vorzugsweise in einem Verdünnungsmittel wie Dimethylsulfoxid, N,N-Dimethylformamid, Hexamethyl-phosphorsäuretriamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindemittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diaza-bicyclo[2,2,2]-octan (DABCO), überschüssiges Piperazinon (III) oder 1,8-Diazabicyclo[5,4,0]undec-7-en (DBU).

Die Reaktionstemperaturen können in einem grösseren Bereich variiert werden. Im allgemeinen arbeitet man zwischen etwa 20 und 200 °C, vorzugsweise zwischen 80 und 180 °C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemässen Verfahrens setzt man auf 1 Mol der Carbonsäure

(II) 1 bis 3 Mol, vorzugsweise 1 bis 1,5 Mol des Piperazinons (III) ein.

Als neue Wirkstoffe seien ausser den als Beispiele aufgeführten Verbindungen im einzelnen genannt:

1-Cyclopropyl-6-fluor-1,4-dihydro-7-(4-methyl-3-oxo-1-piperazinyl)-4-oxo-3-chinolincarbonsäure,

1-Cyclopropyl-7-(4-ethyl-3-oxo-1-piperazinyl)-6-fluor-1,4-dihydro-4-oxo-3-chinolincarbonsäure,

1-Cyclopropyl-6,8-difluor-1,4-dihydro-7-(4-methyl-3-oxo-1-piperazinyl)-4-oxo-3-chinolincarbonsäure,

1-Cyclopropyl-6,8-difluor-1,4-dihydro-7-(4-methyl-3-oxo-1-piperazinyl)-4-oxo-3-chinolincarbonsäure und

1-Cyclopropyl-7-(4-ethyl-3-oxo-1-piperazinyl)-6,8-difluor-1,4-dihydro-4-oxo-3-chinolincarbonsäure.

Herstellungsbeispiele

Herstellung der Ausgangsprodukte II

Beispiel A

1-Cyclopropyl-6,7,8-trifluor-1,4-dihydro-4-oxo-3-chinolincarbonsäure

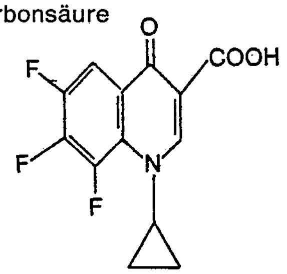

24,3 g Magnesiumspäne werden in 50 ml wasserfreiem Ethanol suspendiert. Man versetzt mit 5 ml Tetrachlorkohlenstoff und tropft, wenn die Reaktion in Gang gekommen ist, ein Gemisch von 160 g Malonsäurediethylester, 100 ml abs. Ethanol und 400 ml wasserfreiem Toluol bei 50–60 °C zu. Dann wird noch 1 Stunde auf 50–60 °C erhitzt, mit Trockeneis/Aceton auf 5 °C bis −10 °C gekühlt und bei dieser Temperatur eine Lösung von 212,5 g 2,3,4,5-Tetrafluorbenzoylchlorid in 80 ml abs. Toluol langsam zugetropft. Man rührt 1 Stunde bei 0 bis −5 °C, lässt über Nacht auf Raumtemperatur kommen und lässt unter Eiskühlung ein Gemisch von 400 ml Eiswasser und 25 ml konz. Schwefelsäure zulaufen. Die Phasen werden getrennt und zweimal mit Toluol nachextrahiert. Die vereinigten Toluollösungen werden mit gesättigter NaCl-Lösung gewaschen, mit $Na_2SO_4$ getrocknet und das Lösungsmittel im Vakuum abgezogen. Man erhält 335 g 2,3,4,5-Tetra-fluorbenzoylmalonsäurediethylester als Rohprodukt.

Eine Emulsion von 284,8 g rohem 2,3,4,5-Tetrafluorbenzoyl-malonsäurediethylester in 300 ml Wasser wird mit 0,3 g p-Toluolsulfonsäure versetzt. Man erhitzt unter gutem Rühren 5 Stunden zum Sieden, extrahiert die erkaltete Emulsion mehrmals mit Methylenchlorid, wäscht die vereinigten Methylenchlorid-Lösungen einmal mit gesättigter NaCl-Lösung, trocknet mit $Na_2SO_4$ und destilliert das Lösungsmittel im Vakuum ab. Die Fraktionierung des Rückstandes im Feinvakuum liefert 160,2 g 2,3,4,5-Tetrafluorbenzoyl-essigsäureethylester vom Siedepunkt 100–110 °C/0,09–0,1 mbar. Schmelzpunkt 47–49 °C.

Ein Gemisch von 110,7 g 2,3,4,5-Tetrafluorbenzoylessigsäureethylester, 93,5 g Orthoameisensäureethylester und 107 g Essigsäureanhydrid wird 2 Stunden auf 150 °C erhitzt. Dann werden im Wasserstrahlvakuum und zuletzt im Hochvakuum bei einer Badtemperatur von 120 °C die flüchtigen Bestandteile abdestilliert. Zurück bleiben 123,9 g roher 2-(2,3,4,5-Tetrafluorbenzoyl)-3-ethoxy-acrylsäureethylester. Er ist genügend rein für die weiteren Umsetzungen.

Eine Lösung von 123,9 g 2-(2,3,4,5-Tetrafluorbenzoyl)-3-ethoxyacrylsäureethylester in 250 ml Ethanol wird unter Eiskühlung und Rühren tropfenweise mit 23,2 g Cyclopropylamin versetzt. Wenn die exotherme Reaktion abgeklungen ist, wird noch 1 Stunde bei Raumtemperatur gerührt, das Lösungsmittel im Vakuum abgezogen und der Rückstand aus Cyclohexan/Petrolether umkristallisiert. Man erhält 115 g

2-(2,3,4,5-Tetrafluorbenzoyl)-3-cyclopropylamino-acrylsäureethylester

vom Schmelzpunkt 63–65 °C.

Eine Lösung von 107,8 g

2-(2,3,4,5-Tetrafluorbenzoyl)-3-cyclopropylamino-acrylsäureethylester

in 400 ml wasserfreiem Dimethylformamid wird mit 21,2 g Natriumfluorid versetzt. Dann wird 2 Stunden unter Rückfluss gerührt und das Reaktionsgemisch heiss auf Eis gegossen. Der Niederschlag wird abgesaugt, mit Wasser gut gewaschen und im Vakuum über Calciumchlorid bei 100 °C getrocknet. Man erhält 91,2 g

1-Cyclopropyl-6,7,8-trifluor-1,4-dihydro-4-oxo-3-chinolincarbonsäureethylester

vom Schmelzpunkt 167–168 °C.

Ein Gemisch von 94 g

1-Cyclopropyl-6,7,8-trifluor-1,4-dihydro-4-oxo-3-chinolincarbonsäureethylester,

600 ml Eisessig, 450 ml Wasser und 70 ml konz. Schwefelsäure wird 1,5 Stunden auf Rückfluss erhitzt. Dann giesst man die heisse Suspension auf Eis, saugt den Niederschlag ab, wäscht gut mit Wasser nach und trocknet im Vakuum bei 100 °C. Man erhält auf diese Weise 88,9 g reine

1-Cyclopropyl-6,7,8-trifluor-1,4-dihydro-4-oxo-3-chinolincarbonsäure

($X = R^2 = F$) vom Schmelzpunkt 228–230 °C (Z).

Beispiel B

1-Cyclopropyl-6,7-difluor-1,4-dihydro-4-oxo-3-chinolincarbonsäure

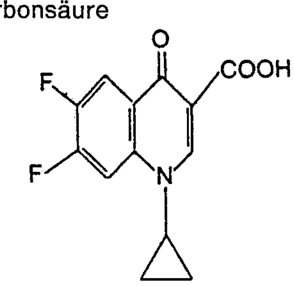

Ausgehend von 2,3,5-Trifluorbenzoylfluorid verfährt man analog Beispiel A, wobei folgende Stufen durchlaufen werden:

2,3,5-Trifluorbenzoyl-essigsäurediethylester (Siedepunkt = 92–95°/0,5 mbar; Schmelzpunkt 53–55 °C) → 2-(2,4,5-Trifluorbenzoyl)-3-cyclopropyl-aminoacrylsäureethylester (Öl) → 1-Cyclopropyl-6,7-difluor-1,4-dihydro-4-oxo-3-chinolincarbonsäureethylester (Schmelzpunkt: 230–233 °C) → 1-Cyclopropyl-6,7-difluor-1,4-dihydro-4-oxo-3-chinolincarbonsäure (Schmelzpunkt: 302–303 °C unter Zersetzung).

Beispiel 1

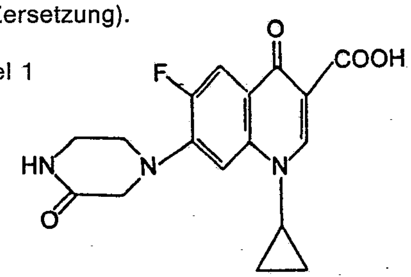

5,3 g (20 mmol) 1-Cyclopropyl-6,7-difluor-1,4-dihydro-4-oxo-3-chinolincarbonsäure werden in 50 ml DMSO vorlegt und mit 2,4 g (24 mmol) 2-Piperazinon und 4,4 g (40 mmol) 1,4-Diazabicyclo[2,2,2]octan 1 Stunde auf 130 °C erhitzt. Nach dem Abkühlen wird die Suspension mit 2 n-Salzsäure auf pH 5 eingestellt, mit 50 ml Wasser versetzt, der Niederschlag abgesaugt, mit Wasser und Methanol gewaschen und anschliessend noch in 50 ml Methanol aufgekocht. Man isoliert 5 g (72% der Theorie)

1-Cyclopropyl-6-fluor-1,4-dihydro-4-oxo-7-(3-oxo-1-piperazinyl)-3-chinolincarbonsäure

vom Schmelzpunkt 342–348 °C (Zersetzung) und einem Gehalt von 97% (HPLC). Massenspektrum: m/e 345 ($M^+$), 301 (94%, $M^+-CO_2$), 231 ($M^+-C_3H_4NO$), 202, 44 (100%, $CO_2$).

NMR ($CF_3COOH$ = δ 1,45 und 1,7 breit (4H im Cyclopropyl-Rest), 3,9 und 4,0 breit (5H, N–CH im Cyclopropyl-Rest, $N-CH_2CH_2-N$), 4,6 breit (2H, $CO-CH_2-N$), 7,85 d (1H, an C–8), 8.28 d (1H, an C–5), 9,3 s (1H, an C–2).

Durch Lösen der Säure in einer äquivalenten Menge 5%iger Natronlauge und Eindampfen der erhaltenen Lösung erhält man das entsprechende Natriumsalz. Es zersetzt sich oberhalb 300 °C.

Führt man die entsprechende Umsetzung mit

7-Chlor-1-cyclopropyl-6-fluor-1,4-dihydro-4-oxo-3-chinolincarbonsäure

bei 120 °C während 16 Stunden durch, dann isoliert man

1-Cyclopropyl-6-fluor-1,4-dihydro-4-oxo-7-(3-oxo-1-piperazinyl)-3-chinolincarbonsäure

in 12% Ausbeute.

Beispiel 2

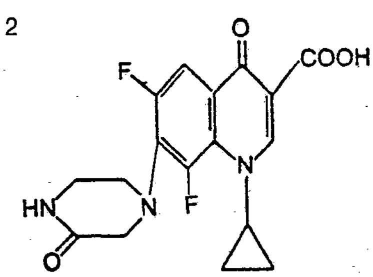

Man setzt die Verbindung aus Beispiel A analog Beispiel 1 mit 2-Piperazinon um und isoliert

1-Cyclopropyl-6,8-difluor-1,4-dihydro-4-oxo-7-(3-oxo-1-piperazinyl)-3-chinolincarbonsäure

vom Schmelzpunkt 322–324 °C (unter Zersetzung) in 58% Ausbeute.

Beispiele für eine erfindungsgemässe Tablette

Jede Tablette enthält:

| | |
|---|---|
| Vebindung des Beispiels | 291,5 mg |
| Mikrokristalline Cellulose | 27,5 mg |
| Maisstärke | 36,0 mg |
| Poly-(1-vinyl-2-pyrrolidon) unlöslich | 15,0 mg |
| Hochdisperses Siliciumdioxid | 2,5 mg |
| Magnesiumstearat | 2,5 mg |
| | 375,0 mg |

Die Lackhülle enthält:

| | |
|---|---|
| Poly-(O-hydroxypropyl)-O-methyl)-cellulose 15 cp (Hydroxypropyl Methylcellulose USP) | 3,9 mg |
| Macrogol 4000 rec. INN (Polyethylenglykole DAB) | 1,3 mg |
| Titan(IV)-oxid (Titanium Dioxide BP) | 1,3 mg |
| | 6,5 mg |

Die erfindungsgemässen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemässen Verbindungen sind gegen ein sehr breites Spektrum von Mikroorganismen wirksam. Mit ihrer Hilfe können gramnegative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemässen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Gemische der folgenden Erreger verursacht werden:

Micrococcaceae, wie Staphylokokken, z.B. Staphylococcus aureus, Staph. Epidermidis, (Staph.

= Staphylococcus); Lactobacteriaceae, wie Streptokokken, z. B. Streptococcus pyogenes, α- bzw. β-hämolysierende Streptokokken, nicht-γ-hämolysierende Streptokokken, Enterokokken und Diplococcus pneumoniae (Pneumokokken) (Str. = Strepococcus); Enterobacteriaceae, wie Escherichiae-Bakterien der Coli-Gruppe; Escherichia-Bakterien, z. B. Escherichia coli, Enterobacter-Bakterien, z. B. E. aerogenes, E. cloacae (E. = Enterobacter), Klebsiella-Bakterien, z. B. pneumoniae (K. = Klebsiella), Serratia, z. B. Serratia marcescens, Proteae-Bakterien der Proteus-Gruppe: Proteus, z. B. Pr. vulgaris, Pr. morganii, Pr. rettgeri, Pr. mirabilis (Pr. = Proteus); Pseudomonadaceae, wie Pseudomonas-Bakterien, z. B. Ps. aeruginosa (Ps. = Pseudomonas); Bacteroidaceae, wie Bacteroides-Bakterien, z. B. Bacteroides fragilis; Mykoplasmen, z. B. Mycoplasma pneumoniae, ausserdem Mykobakterien, z. B. Mycobacterium tuberculosis, Mycobacterium leprae und atypische Mykobakterien.

Die obige Aufzählung von Erregern ist lediglich beispielhaft und keineswegs beschränkend aufzufassen.

Als Krankheiten, die durch die erfindungsgemässen Verbindungen verhindert, gebessert und/oder geheilt werden können, seien beispielsweise genannt:

Erkrankungen der Atmungswege und des Rachenraumes:

Otitis; Pharyngitis; Pneumonie; Peritonitis; Pyelonephritis; Cystitis; Endocarditis; Systeminfektionen; Bronchitis, Arthritis; lokale Infektionen, septische Erkrankungen.

Zur Erfindung gehören pharmazeutische Zubereitungen, die neben nicht-toxischen, inerten pharmazeutisch geeigneten Trägerstoffen einen oder mehrere erfindungsgemässe Verbindungen enthalten oder die aus einem oder mehreren erfindungsgemässen Wirkstoffen bestehen, sowie Verfahren zur Herstellung dieser Zubereitungen.

Zur Erfindung gehören auch pharmazeutische Zubereitungen in Dosierungseinheiten. Dies bedeutet, dass die Zubereitung in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder ½, ⅓ oder ¼ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzukker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumcarbonat, (e) Lösungsverzögerer, z. B. Paraffin und (f) Resorptionsbeschleuniger, z. B. quarternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden, Überzügen und Hüllen versehen sein und auch so zusammengesetzt sein, dass sie den oder die Wirkstoffe nur oder bevorzugt in einem bestimmten Teil des Intestinaltraktes gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z. B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnussöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süssmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können ausser den erfindungsgemässen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemässen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemässen Wirkstoffe, vorzugsweise in Mengen von etwa 1 bis etwa 250, insbesondere 3 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführten Wirkstoffmenge überschritten werden muss. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

Aus J. Med. Chem. 1358 (1980), ist bereits bekannt, dass 1-Ethyl-6-fluor-1,4-dihydro-4-oxo-7-(1-piperazinyl)-3-chinolincarbonsäure (Norfloxacin) antibakterielle Eigenschaften besitzt. Die erfindungsgemässen Verbindungen sind jedoch dem Norfloxacin überlegen.

Die nachstehenden Tabellen 1 und 2 zeigen beispielhaft die MHK-Werte der Verbindung des Beispiels 1 im Vergleich mit Norfloxacin.

Tabelle 1:
Agardilutionstest / Isosensitestmedium

| | MHK (mcg/ml) Staph. aur. FK422 | Staph. 1756 | Staph 133 |
|---|---|---|---|
| Verbindung des Beispiels 1 | 0,5 | 0,25 | 0,5 |
| Norfloxacin | 2 | 2 | 1 |

Tabelle 2:
Mikrotiterassay/Urin (80% gepoolter Humanurin, 20% Isosensitestmedium)

| Stamm | MHK mcg/ml Urin pH 5,7 | pH 7,1 |
|---|---|---|
| E. coli C14 | | |
| Norfloxacin | 4 | 0,5 |
| Verbindung des Beispiels 1 | 0,25 | 0,25 |
| Staph. 25151 | | |
| Norfloxacin | 4 | 4 |
| Verbindung des Beispiels 1 | 0,25 | 0,5 |

## Patentansprüche

1. 1-Cyclopropyl-6-fluor-1,4-dihydro-4-oxo-7-(3-oxo-1-piperazinyl)-3-chinolincarbonsäuren der Formel (I)

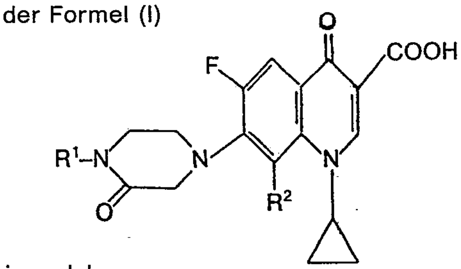

(I),

in welcher

$R^1$ Wasserstoff, Methyl oder Ethyl und

$R^2$ Wasserstoff oder Fluor bedeuten,

sowie deren pharmazeutisch verwendbaren Hydrate, Alkali- und Erdalkalisalze.

2. 1-Cyclopropyl-6-fluor-1,4-dihydro-4-oxo-7-(3-oxo-1-piperazinyl)-3-chinolincarbonsäure.

3. 1-Cyclopropyl-6,8-difluor-1,4-dihydro-4-oxo-7-(3-oxo-1-piperazinyl)-3-chinolincarbonsäure.

4. 1-Cyclopropyl-6-fluor-1,4-dihydro-4-oxo-7-(3-oxo-1-piperazinyl)-3-chinolincarbonsäuren der Formel (I)

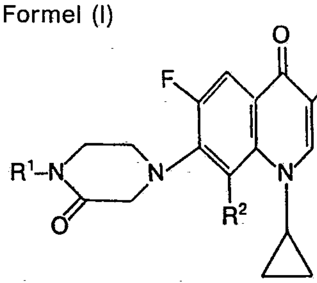

(I),

in der

$R^1$ Wasserstoff, Methyl oder Ethyl oder

$R^2$ Wasserstoff oder Fluor bedeuten,

sowie deren pharmazeutisch verwendbaren Hydrate, Alkali- und Erdalkalisalze zur Verwendung bei der Behandlung von Erkrankungen.

5. Arzneimittel enthaltend 1-Cyclopropyl-6-fluor-1,4-dihydro-4-oxo-7-(3-oxo-1-piperazinyl)-3-chinolincarbonsäuren der Formel (I)

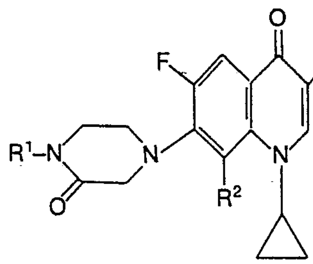

(I),

in welcher

$R^1$ Wasserstoff, Methyl oder Ethyl und

$R^2$ Wasserstoff oder Fluor bedeuten,

sowie deren pharmazeutisch verwendbaren Hydrate, Alkali- und Erdalkalisalze.

6. Verfahren zur Herstellung eines Arzneimittels, dadurch gekennzeichnet, dass man 1-Cyclopropyl-6-fluor-1,4-dihydro-4-oxo-7-(3-oxo-1-piperazinyl)-3-chinolincarbonsäuren der Formel (I)

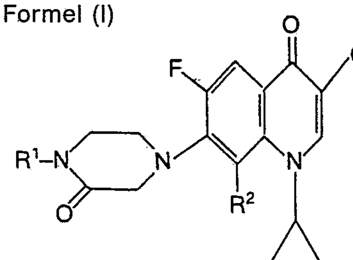

(I),

in welcher

$R^1$ Wasserstoff, Methyl oder Ethyl und

$R^2$ Wasserstoff oder Fluor bedeuten,

sowie deren pharmazeutisch verwendbare Hydrate, Alkali- und Erdalkalisalze mit inerten, nichttoxischen, pharmazeutisch geeigneten Trägerstoffen vermischt.

7. Verfahren zur Herstellung von 1-Cyclopropyl-6-fluor-1,4-dihydro-4-oxo-7-(3-oxo-1-piperazinyl)-3-chinolincarbonsäuren der Formel (I)

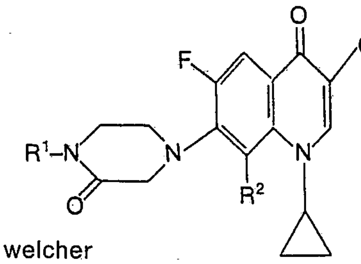

(I),

in welcher

$R^1$ Wasserstoff, Methyl oder Ethyl und

$R^2$ Wasserstoff oder Fluor bedeuten,

dadurch gekennzeichnet, dass man eine Verbindung der Formel (II)

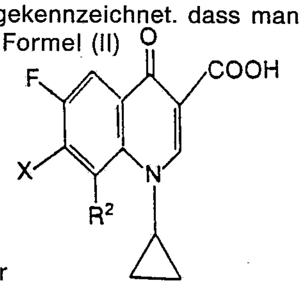

(II)

in welcher

$R^2$ die oben angegebene Bedeutung hat und

X für Chlor, Brom oder Fluor steht,

mit einem 2-Piperazinon der Formel (III)

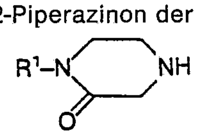

(III)

in welcher

$R^1$ die oben angegebene Bedeutung hat, umsetzt.

8. Verfahren nach Anspruch 8, dadurch gekennzeichnet, dass man die Umsetzung in Dimethylsulfoxid, N,N-Dimethylformamid, Hexamethylphosphorsäuretrisamid, Sulfolan, Wasser, Methanol, Ethanol, n-Propanol, Isopropanol, Glykolmonomethylether oder Pyridin durchführt.

9. Verfahren nach den Ansprüchen 8 und 9, dadurch gekennzeichnet, dass man die Reaktion bei Temperaturen zwischen 20 und 200 °C und bei Drucken zwischen 1 und 10 bar durchführt.

**Claims**

1. 1-Cyclopropyl-6-fluoro-1,4-dihydro-4-oxo-7-(3-oxo-1-piperazinyl)-3-quinolinecarboxylic acids of the formula (I)

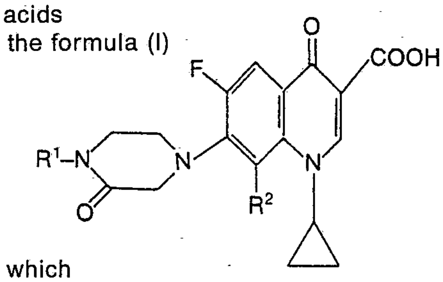

(I)

in which

$R^1$ denotes hydrogen, methyl or ethyl, and

$R^2$ denotes hydrogen or fluorine,

and their pharmaceutically utilizable hydrates, alkali metal and alkaline earth metal salts.

2. 1-Cyclopropyl-6-fluoro-1,4-dihydro-4-oxo-7-(3-oxo-1-piperazinyl)-3-quinolinecarboxylic acid.

3. 1-Cyclopropyl-6,8-difluoro-1,4-dihydro-4-oxo-7-(3-oxo-1-piperazinyl)-3-quinolinecarboxylic acid.

4. 1-Cyclopropyl-6-fluoro-1,4-dihydro-4-oxo-7-

(3-oxo-1-piperazinyl)-3-quinolinecarboxylic acids
of the formula (I)

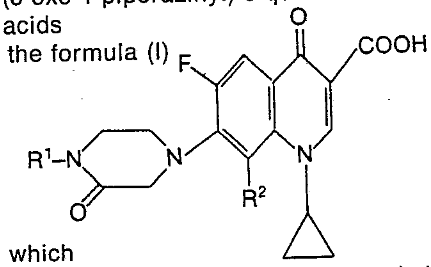

(I)

in which
$R^1$ denotes hydrogen, methyl or ethyl, or
$R^2$ denotes hydrogen or fluorine,
and their pharmaceutically utilizable hydrates, alkali metal and alkaline earth metal salts, for use in the treatment of illness.

5. Medicament containing
1-cyclopropyl-6-fluoro-1,4-dihydro-4-oxo-7-(3-oxo-1-piperazinyl)-3-quinolinecarboxylic acids
of the formula (I)

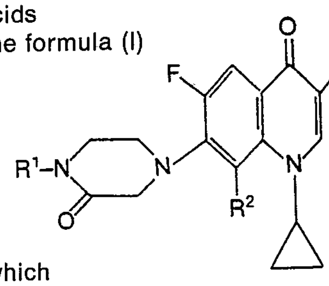

(I)

in which
$R^1$ denotes hydrogen, methyl or ethyl, and
$R^2$ denotes hydrogen or fluorine,
and their pharmaceutically utilizable hydrates, alkali metal and alkaline earth metal salts.

6. Process for the preparation of a medicament, characterized in that
1-cyclopropyl-6-fluoro-1,4-dihydro-4-oxo-7-(3-oxo-1-piperazinyl)-3-quinolinecarboxylic acids
of the formula (I)

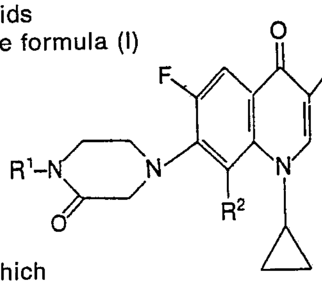

(I)

in which
$R^1$ denotes hydrogen, methyl or ethyl, and
$R^2$ denotes hydrogen or fluorine,
and their pharmaceutically utilizable hydrates, alkali metal and alkaline earth metal salts, are mixed with inert, non-toxic, pharmaceutically suitable vehicles.

7. Process for the preparation of
1-cyclopropyl-6-fluoro-1,4-dihydro-4-oxo-7-(3-oxo-1-piperazinyl)-3-quinolinecarboxylic acids
of the formula (I)

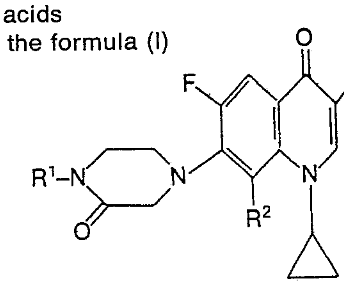

(I)

in which
$R^1$ denotes hydrogen, methyl or ethyl, and
$R^2$ denotes hydrogen or fluorine,
characterized in that a compound of the formula (II)

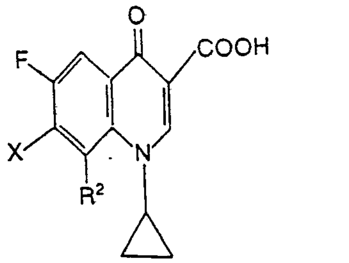

(II)

in which
$R^2$ has the abovementioned meaning and
X represents chlorine, bromine or fluorine, is reacted with a 2-piperazinone of the formula (III)

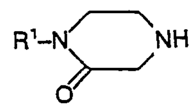

(III)

in which
$R^1$ has the abovementioned meaning.

8. Process according to Claim 7, characterized in that the reaction is carried out in dimethylsulphoxide, N,N-dimethylformamide, hexamethylphosphoric acid triamide, sulpholane, water, methanol, ethanol, n-propanol, isopropanol or glycol monomethyl ether or pyridine.

9. Process according to Claims 7 and 8, characterized in that the reaction is carried out at temperatures between 20 and 200 °C and under pressure between 1 and 10 bar.

**Revendications**

1. Acides
1-cyclopropyl-6-fluoro-1,4-dihydro-4-oxo-7-(3-oxo-1-pipérazinyl)-3-quinoléinecarboxyliques
de formule (I)

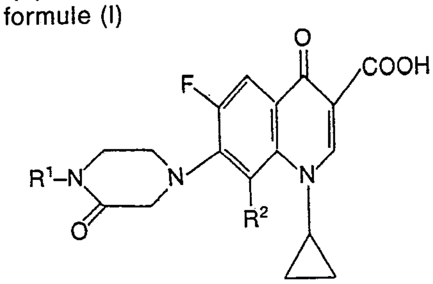

(I)

dans laquelle
$R^1$ représente l'hydrogène, méthyle ou éthyle et
$R^2$ représente l'hydrogène ou le fluor,
ainsi que leurs hydrates et leurs sels alcalins et alcalino-terreux utilisables en pharmacie.

2. L'acide
1-cyclopropyl-6-fluoro-1,4-dihydro-4-oxo-7-(3-oxo-1-pipérazinyl)-3-quinoléinecarboxylique.

3. L'acide
1-cyclopropyl-6,8-difluoro-1,4-dihydro-4-oxo-7-(3-oxo-1-pipérazinyl)-3-quinoléinecarboxylique.

4. Des acides
1-cyclopropyl-6-fluoro-1,4-dihydro-4-oxo-7-(3-oxo-1-pipérazinyl)-3-quinoléinecarboxyliques
de formule (I)

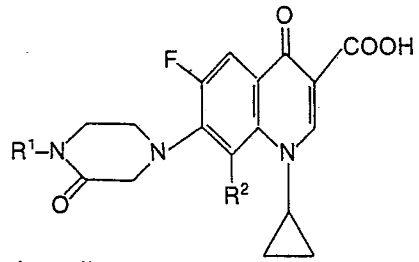

(I)

dans laquelle
$R^1$ représente l'hydrogène, méthyle ou éthyle et
$R^2$ représente l'hydrogène ou le fluor,
ainsi que leurs hydrates et leurs sels alcalins et alcalino-terreux utilisables en pharmacie, pour l'utilisation dans le traitement de maladies.

5. Médicament contenant des acides 1-cyclopropyl-6-fluoro-1,4-dihydro-4-oxo-7-(3-oxo-1-pipérazinyl)-3-quinoléinecarboxyliques de formule (I)

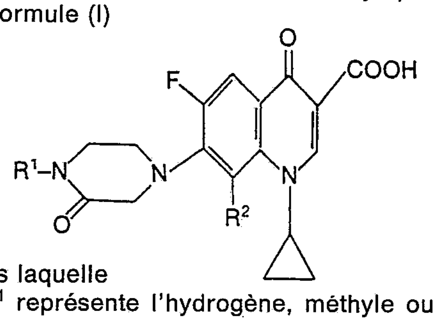

(I)

dans laquelle
$R^1$ représente l'hydrogène, méthyle ou éthyle et,
$R^2$ représente l'hydrogène ou le fluor,
ainsi que leurs hydrates et leurs sels alcalins et alcalino-terreux utilisables en pharmacie.

6. Procédé pour la fabrication d'un médicament, caractérisé en ce qu'on mélange des acides 1-cyclopropyl-6-fluoro-1,4-dihydro-4-oxo-7-(3-oxo-1-pipérazinyl)-3-quinoléinecarboxyliques de formule (I)

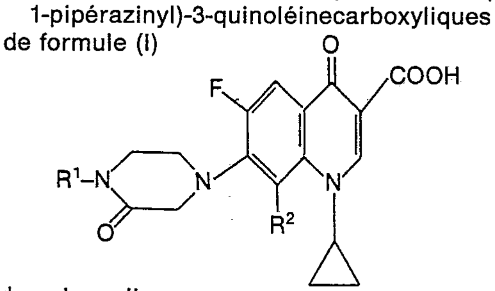

(I)

dans laquelle
$R^1$ représente l'hydrogène, méthyle ou éthyle et
$R^2$ représente l'hydrogène ou le fluor,
ainsi que leurs hydrates et leurs sels alcalins et alcalino-terreux utilisables en pharmacie, avec des supports inertes, non toxiques, acceptables en pharmacie.

7. Procédé pour la fabrication d'acides 1-cyclopropyl-6-fluoro-1,4-dihydro-4-oxo-7-(3-oxo-1-pipérazinyl)-3-quinoléinecarboxyliques de formule (I)

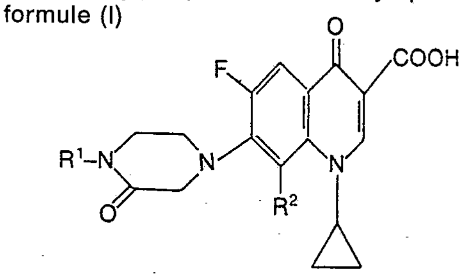

(I)

dans laquelle
$R^1$ représente l'hydrogène, méthyle ou éthyle et
$R^2$ représente l'hydrogène ou le fluor,
caractérisé en ce qu'on fait réagir un composé de formule (II)

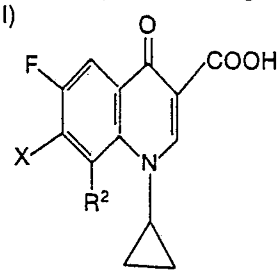

(II)

dans laquelle
$R^2$ a la signification indiquée ci-dessus et
X représente le chlore, le brome ou le fluor
avec une 2-pipérazinone de formule (III)

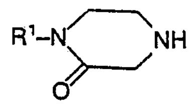

(III)

dans laquelle
$R^1$ a la signification indiquée ci-dessus.

8. Procédé selon la revendication 7, caractérisé en ce qu'on met en œuvre la réaction dans le diméthylsulfoxyde, le N,N-diméthylformamide, l'hexaméthyltriamide phosphorique, le sulfolanne, l'eau, le méthanol, l'éthanol, le n-propanol, l'isopropanol, l'éther monométhylique de glycol ou la pyridine.

9. Procédé selon les revendications 8 et 9, caractérisé en ce qu'on met en œuvre la réaction et des températures comprises entre 20 et 200 °C sous des pressions comprises entre 1 et 100 bar.